# EUROPEAN PATENT APPLICATION

(11) **EP 4 498 076 A1**
(43) Date of publication of application: **29.01.2025**
(21) Application number: 23306278.5
(22) Date of filing: 24.07.2023
(51) Int. Cl.: G01N 27/12, G01N 33/00

(54) **MICROSENSOR FOR THE MEASUREMENT OF HYDROGEN**

(71) Applicant: Centre National de la Recherche Scientifique, 75016 Paris (FR); Université d'Aix-Marseille, 13007 Marseille (FR)
(72) Inventor: FIORIDO, Tomas, 13800 ISTRES (FR); OCCELLI, Clément, 69003 LYON (FR); SEGUIN, Jean-Luc, 13100 AIX-EN-PROVENCE (FR)
(74) Representative: Cabinet Nony

(57) **Abstract**

A microsensor for the measurement of hydrogen, especially hydrogen injected into natural gas networks, comprising at least:
- a substrate, preferably made of oxidized silicon,
- a heating track, and
- a resistive sensitive track made at least from a palladium-gold alloy, the amount of gold in said sensitive track being equal to or smaller than 20%.

## Description

### Technical field

The invention relates to microsensors for the measurement of hydrogen, especially hydrogen injected into natural gas networks.

A growth in renewable electricity generation has undeniably occurred. Solutions for storing surplus production have now to be found and optimized.

Power to gas (P2G) is based on the production of green hydrogen by electrolysis of water and its storage in the natural gas network. However, because of its high permeability through many metals and its reactivity, hydrogen can weaken gas pipes, and rapid variations in the level of hydrogen injected can disrupt the operation of turbines and burners. Real-time measurement of hydrogen content in the network is therefore essential to guard against risky situations.

### Prior art

The article of Darmadi et al., "Rationally Designed PdAuCu Ternary Alloy Nanoparticles for intrinsically deactivation-resistant ultrafast plasmonic hydrogen sensing", ACS Sensors 2019, vol 4, pages 1424-1432, describes an optical hydrogen sensor for obtaining hydrogen detection measurements in air, made of a palladium, gold and copper alloy. This optical sensor is fabricated by layer-by-layer deposition of suitable amounts of Cu, Au and Pd via an electrolysis system, the amount of gold being approximatively equal to 25%.

The article of Nugroho et al., "Universal Scaling and Design Rules of Hydrogen-Induced optical properties in Pd and Pd-alloy nanoparticles", ACS Nano 2018, vol 12, pages 9903-9912, does not deal with resistive sensors, but describes sensors for plasmonic hydrogen sensing, describing hydrogen-induced optical properties in Pd nanoparticles and Pd alloy nanoparticles.

The article of Wadell et al., "Hysteresis-Free Nanoplasmonic Pd-Au Alloy Hydrogen sensors", NANO letters 2015, vol 15, pages 3563-3570, also discloses nanoplasmonic Pd-Au alloy hydrogen sensors for measuring hydrogen in air.

Patent application CN 108872314 describes a piezoelectric hydrogen sensor comprising an encapsulation shell and a hydrogen sensor piezoelectric chip, said chip consisting of a metal palladium film, a conductive substrate and a ZnO nano material array, the chip being arranged inside the encapsulation shell, the top and the bottom of the encapsulation shell being provided with two gold electrodes.

Patent application JP 2003-279522 discloses a hydrogen detecting apparatus provided with an insulating substrate, sensing membranes 30 and 31 having Pd as a main component and provided on one surface of the insulating substrate, and oxidation-resistive membranes.

Patent application JP 2002-214190 describes a constant-potential electrolytic hydrogen sensor comprises a working electrode for chemically reacting to a gas to be detected, a counter electrode opposed to the working electrode, and a reference electrode for controlling the potential of the working electrode.

An electrochemical sensor is also known from patent application CN 104634837.

The article of the inventors, *"*PdAu Based Resistive Hydrogen Sensor in Anaerobic Environment", November 2021, Conference SENSORCOMM 2021, describes a single macro-sensor and a continuous layer for physico-chemical analysis.

The article of Gong et al., "MEMS-based resistive hydrogen sensor with high performance using a palladium-gold alloy thin film", Journal of alloys and compounds 930 (2023), describes a resistive microsensor for the measurement of hydrogen, with a resistive sensitive track made of a palladium-gold alloy, with an amount of gold equal to 33%. The detection kinetics of this sensor are particularly slow, in relation to grain size, temperature and gold content, because of the fact that the excessive gold amount competes with the dissociation and adsorption sites on the palladium surface.

Hydrogen sensors currently known cannot efficiently operate in an environment devoid of oxygen, humidity or containing numerous chemical compounds.

There is thus a need to further improve sensors for the measurement of hydrogen.

The present invention notably seeks to meet this need.

### Disclosure of the invention

### Microsensor

One subject of the invention is a microsensor for the measurement of hydrogen, especially hydrogen injected into natural gas networks, comprising at least:
- a substrate, preferably made of oxidized silicon,
- a heating track, and
- a resistive sensitive track made at least from a palladium-gold alloy, the amount of gold in said sensitive track being equal to or smaller than 20%.

The microsensor according to the invention provides an efficient tool for measuring hydrogen in an anaerobic environment and at room temperature. Moreover, it is quite simple to manufacture since the use of a hydrogen-inert gold track for heating allows avoiding burying it under a protective layer.

Such a microsensor is capable of operating at different temperatures, and in low-temperature operations, like 50°C. The microsensor allows hydrogen selectivity compared with natural gas components, short response times, namely a few seconds, the adaptation to different hydrogen concentration ranges by modifying the alloy composition, a power consumption less than 1W, and the detection of concentration fluctuations and brief exposures

The microsensor according to the invention offers a satisfactory detection kinetics, faster than the ones obtained with the known sensors, thanks especially to the grain size and the small gold amount in the sensitive track. An excessive gold amount indeed competes with the dissociation and adsorption sites on the palladium surface.

The microsensor of the invention is a resistive sensor, that is to say that hydrogen is detected by reading the variation in resistance of the sensitive track.

The microsensor according to the invention is advantageously dedicated to the measurement of hydrogen in anaerobic conditions, that is to say in the absence of oxygen.

The microsensor according to the invention may be used for:
- the measurement of hydrogen injected into natural gas,
- the monitoring of hydrogen tanks in future vehicles,
- the detection of hydrogen leaks in future service stations,
- medical applications, like the detection of dysbiosis by measuring hydrogen concentration in breath,
- the recirculation of hydrogen in fuel cells and methanation reactors, and/or
- the control of chemical processes, especially ammonia and methanol, and metallurgical processes.

In a preferred embodiment, the total height of the microsensor is comprised between 2 mm and 10 mm, being preferably equal to 4 mm. The total width of the microsensor is advantageously comprised between 2 mm and 10 mm, being preferably equal to 4 mm.

The amount of gold in the sensitive track may be equal to or smaller than 15%, preferably equal to or smaller than 10%, being preferably comprised between 5% and 10%, being preferably equal to 8%.

The amount of gold in said sensitive track may be higher than 5%.

The sensitive track may further contain copper, preferably between 2% and 15%, preferably equal to or smaller than 10%.

The sensitive track may be located inside a circular or ellipsoidal zone formed and heated by the heating track.

The operating temperature of the microsensor is advantageously equal to or smaller than 55°C, being preferably equal to 50°C.

The thickness of the sensitive track may be comprised between 20 nm and 120 nm, being preferably equal to 43 nm.

The thickness of the heating track may be comprised between 100 nm and 200 nm, being preferably equal to 170 nm.

The length of the sensitive track may be comprised between 6 mm and 20 mm, being preferably equal to 10 mm.

The width of the sensitive track may be comprised between 10 µm and 100 µm, being preferably equal to 20 µm.

The sensitive track is advantageously made of an alloy comprising grains, and the grain size of the microsensor, measured by DRX, may be comprised between 10 nm and 30 nm, being preferably equal to 16 nm.

The heating track may be made at least of gold, preferably of pure gold. In this case, there is no need to add a layer inert to hydrogen.

The microsensor according to the invention may further comprise an adherence layer made of chrome for the adherence of the heating and sensitive tracks to the substrate, the thickness of said adherence layer being preferably equal to 5nm.

All the amounts of gold, copper or palladium or any other material are by mass of the total weight.

### Manufacturing method

According to another one of its aspects, the invention also relates to a method for manufacturing a microsensor for the measurement of hydrogen, especially hydrogen injected into natural gas networks, wherein first the heating track and then the sensitive track are deposited on the substrate by cathode sputtering, preferably RF sputtering, the sputtering being preferably performed at a voltage equal to 900 V, the gas used for the sputtering being preferably argon.

The sputtering pressure may be comprised between 2 Pa and 8 Pa, being preferably equal to 6 Pa.

Said manufacturing method may further comprise an annealing step, performed at a temperature of 200°C, during at least 2h, preferably during 4h, and in an atmosphere devoid of oxygen, preferably in azote N₂, preferably in a tubular oven.

### Measuring method

According to another one of its aspects, the invention also relates to a method for measuring hydrogen, especially hydrogen injected into natural gas networks, the method using at least one microsensor to produce an electrical resistance signal representing the amount of hydrogen present, said at least one microsensor comprising at least a substrate, a heating track and a resistive sensitive track made at least from a palladium-gold alloy, the amount of gold in said sensitive track being equal to or smaller than 20%.

The features described for the microsensor applies to the methods and vice and versa.

### Brief description of the drawings

The invention may be better understood upon reading the following detailed description of nonlimiting implementation examples thereof and on studying the appended drawing, in which:
- figure 1 represents a microsensor according to one embodiment of the invention,
- figure 2 illustrates the performances of the microsensor according to the invention, and
- figure 3 illustrates the use of the microsensor according to the invention for measuring hydrogen injected into natural gas.

### Detailed description

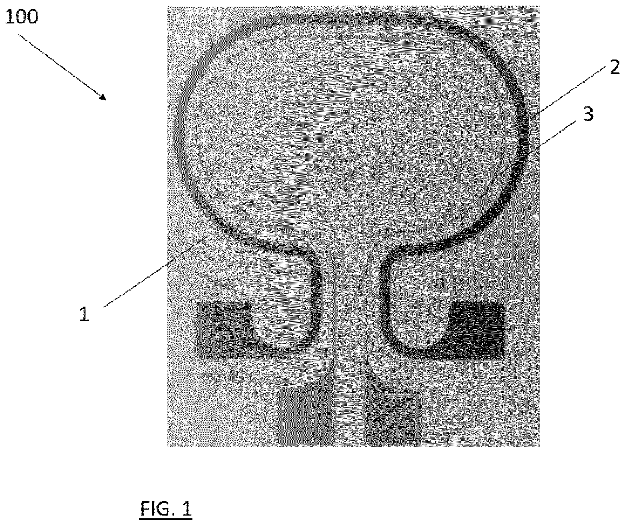

Figure 1 illustrates an example of the microsensor according to the invention.

In this embodiment, a microsensor 100 according to the invention and for the measurement of hydrogen comprises a substrate 1 made of oxidized silicon, a heating track 2 made of pure gold, and a resistive sensitive track 3 made at least from a palladium-gold alloy, the amount of gold in said sensitive track 3 being equal to 8%. In this example, the total height of the microsensor 100 is equal to 4 mm, and its total width is equal to 4 mm. The sensitive track 3 is located inside an ellipsoidal zone formed and heated by the heating track 2.

In this embodiment, the sensitive track 3 further contains copper, between 2% and 15%.

The thickness of the sensitive track 3 is equal to 43 nm in this example, while the thickness of the heating track 2 is equal to 170 nm.

In this embodiment, the length of the sensitive track 3 is equal to 10.3 mm, and the width of the sensitive track 3 is equal to 20 µm. The width of the heating track 2 is equal to 100 µm.

Preferably and in the illustrated embodiment, the operating temperature of the microsensor 100 is equal to 50°C.

The sensitive track 3 is made of an alloy comprising grains, the grain size of the microsensor 100, measured by DRX, is preferably comprised between 10 nm and 30 nm, being for example equal to 16 nm.

The illustrated microsensor 100 further comprises an adherence layer made of chrome for the adherence of the heating and sensitive tracks to the substrate, the thickness of said adherence layer being for example equal to 5nm.

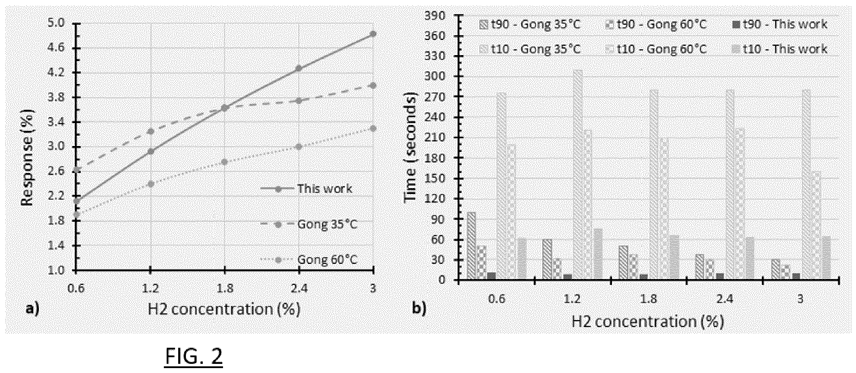

Figure 2 illustrates the performances of the microsensor according to the invention and shows a comparison with the performances of the microsensor described in the article of Gong et al., *"MEMS-based resistive hydrogen sensor with high performance using a palladium-gold alloy thin film",* Journal of alloys and compounds 930 (2023).

Preferably and in the considered example, for manufacturing the microsensor 100 for the measurement of hydrogen, first the heating track 2 and then the sensitive track 3 are deposited on the substrate 1 by cathode RF sputtering, said sputtering being performed at a voltage equal to 900 V, the gas used for the sputtering being argon. The sputtering pressure is equal in this example to 6 Pa.

The method for manufacturing the microsensor 100 further comprises, in the illustrated example, an annealing step, performed at a temperature of 200°C, during 4h, and in an atmosphere devoid of oxygen, in azote N₂, in a tubular oven.

In this example, the microsensor 100 according to the invention is as described in relation to figure 1.

The sensor of Gong incorporates a sensitive track in a PdAu alloy, and a heating track made of pure gold. The sensor also comprises a track made of Pt for acting as a temperature regulation probe and a silicon nitride layer for chemical and thermal insulation. The amount of gold of their sensitive track is however 33%, compared with 8% in the invention. Grain size measured by DRX is 8.4 nm, almost half that of the microsensor according to the invention. A 20 nm thick layer of titanium is used to adhere the various tracks to the substrate, compared with 5 nm of chrome in the invention. The preferred temperature used by Gong is 60°C, compared with 50°C for the tests of the microsensor 100 according to the invention.

The tests reveal that while the response amplitude of the microsensor of the invention for a 3% H₂ exposure is greater than that of the sensor of Gong, with 4.8% vs. 3.3%, the response and return times of the microsensor of the invention are also considerably shorter than those of the sensor of Gong, with 10s/65s vs. 22s/160s.

The detection kinetics of the sensor of Gong are particularly slow compared with the ones obtained with the invention, in relation to grain size, temperature and gold content. One explanation is that the excessive gold content competes with the dissociation and adsorption sites on the palladium surface.

Figures 2.a) and 2.b) compare the performances of the two different sensitive tracks for several pulse exposures to hydrogen. It can be seen that the microsensor according to the invention has a higher response amplitude and therefore a better sensitivity than the sensor of Gong for operation at 60°C, over the entire H₂ concentration range. For operation at 35°C, the response amplitude of the microsensor according to the invention is higher from 1.8% H₂. With respect to response and return times, the microsensor of the invention is systematically faster than the sensor described in Gong, whatever the concentration or operating temperature. For example, for exposure to 0.6% H₂, the response and return times of the sensor of Gong are respectively 100s/275s at 35°C and 50s/200s at 60°C, whereas they are only 11s/61s (at 50°C) for the microsensor of the invention. For exposure to 3% H₂, the microsensor according to the invention still has the shortest detection kinetics.

Finally, a look at the signal of the sensor of Gong shows that its resistance decreases when exposed to hydrogen. This behavior is not unlike that of some of the known macro-sensors, where a partial inverse response can be observed from a gold content of 25.8%, and a total inverse response from 31.6%. As a reminder, the gold content of the Gong sensor's sensitive track is 33%. These results corroborate the phenomenon of inverse response, i.e. the fact that reduced resistance when exposed to hydrogen is linked to the gold content of the layer.

The tests show that the invention is a very efficient instrument for measuring hydrogen, in an anaerobic environment and at room temperature, from 0 to 3% H2. Moreover, it's simpler to manufacture, since it is not necessary to bury it under a protective layer, as a hydrogen-inert gold track is used for heating.

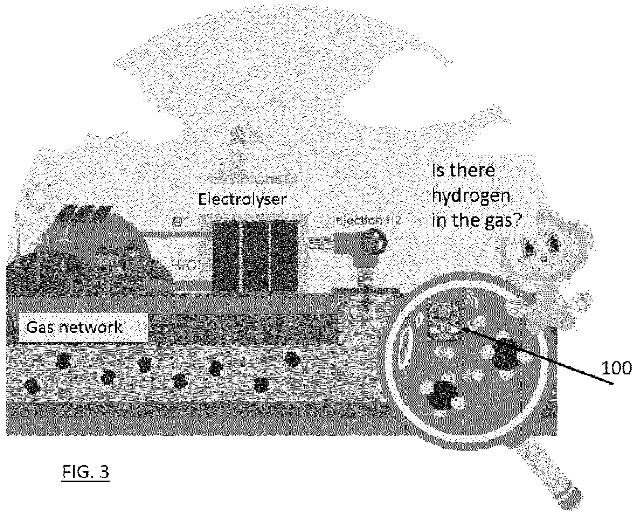

The microsensor 100 according to the invention may be used for measuring hydrogen injected into natural gas networks, as illustrated in figure 3.

The invention is not limited to the examples that have just been described.

For example, other dimensions or other shapes for the tracks may be chosen, and additional components may be used.

## Claims

1. Microsensor for the measurement of hydrogen, especially hydrogen injected into natural gas networks, comprising at least:
- a substrate, preferably made of oxidized silicon,
- a heating track, and
- a resistive sensitive track made at least from a palladium-gold alloy, the amount of gold in said sensitive track being equal to or smaller than 20%.

2. Microsensor as claimed in claim 1, wherein the total height of the microsensor is comprised between 2 mm and 10 mm, being preferably equal to 4 mm.

3. Microsensor as claimed in claim 1 or 2, wherein the total width of the microsensor is comprised between 2 mm and 10 mm, being preferably equal to 4 mm.

4. Microsensor as claimed in any one of the preceding claims, wherein the amount of gold in the sensitive track is equal to or smaller than 15%, preferably equal to or smaller than 10%, being preferably comprised between 5% and 10%, being preferably equal to 8%.

5. Microsensor as claimed in any one of the preceding claims, wherein the sensitive track further contains copper, preferably between 2% and 15%.

6. Microsensor as claimed in any one of the preceding claims, wherein the sensitive track is located inside a circular or ellipsoidal zone formed and heated by the heating track.

7. Microsensor as claimed in any one of the preceding claims, wherein the operating temperature of the microsensor is equal to or smaller than 55°C, being preferably equal to 50°C.

8. Microsensor as claimed in any one of the preceding claims, wherein the thickness of the sensitive track is comprised between 20 nm and 120 nm, being preferably equal to 43 nm.

9. Microsensor as claimed in any one of the preceding claims, wherein the thickness of the heating track is comprised between 100 nm and 200 nm, being preferably equal to 170 nm.

10. Microsensor as claimed in any one of the preceding claims, wherein the length of the sensitive track is comprised between 6 mm and 20 mm, being preferably equal to 10 mm, and the width of the sensitive track is comprised between 10 µm and 100 µm, being preferably equal to 20 µm.

11. Microsensor as claimed in any one of the preceding claims, wherein the sensitive track is made of an alloy comprising grains, the grain size of the microsensor, measured by DRX, is comprised between 10 nm and 30 nm, being preferably equal to 16 nm.

12. Microsensor as claimed in any one of the preceding claims, wherein the heating track is made at least of gold, preferably of pure gold.

13. Microsensor as claimed in any one of the preceding claims, further comprising an adherence layer made of chrome for the adherence of the heating and sensitive tracks to the substrate, the thickness of said adherence layer being preferably equal to 5nm.

14. Method for manufacturing a microsensor for the measurement of hydrogen, especially hydrogen injected into natural gas networks, wherein first the heating track and then the sensitive track are deposited on the substrate by cathode sputtering, preferably RF sputtering, the sputtering being preferably performed at a voltage equal to 900 V, the gas used for the sputtering being preferably argon, the sputtering pressure being preferably comprised between 2 Pa and 8 Pa, being preferably equal to 6 Pa.

15. Method for measuring hydrogen, especially hydrogen injected into natural gas networks, the method using at least one microsensor to produce an electrical resistance signal representing the amount of hydrogen present, said at least one microsensor comprising at least a substrate, a heating track and a resistive sensitive track made at least from a palladium-gold alloy, the amount of gold in said sensitive track being equal to or smaller than 20%.
